# EUROPEAN PATENT SPECIFICATION

(11) **EP 1 370 279 B1**
(45) Date of publication and mention of the grant of the patent: **07.09.2005**
(21) Application number: 01983771.5
(22) Date of filing: 18.10.2001
(51) Int. Cl.: A61K 38/18, A61P 37/00

(54) **USE OF THE LONG PENTRAXIN PTX3 FOR THE PREPARATION OF MEDICAMENT FOR THE PREVENTION AND CURE OF AUTOIMMUNE PATHOLOGIES**
VERWENDUNG VON LANGEM PENTRAXIN PTX3 ZUR HERSTELLUNG EINES ARZNEIMITTELS ZUR VERHÜTUNG UND HEILUNG VON AUTOIMMUNKRANKHEITEN
UTILISATION DE LA PENTRAXINE PTX3 LONGUE POUR LA PREPARATION D'UN MEDICAMENT UTILISE POUR LA PREVENTION ET LE TRAITEMENT DE PATHOLOGIES AUTO-IMMUNES

(30) Priority: 03.11.2000 IT RM20000571
(43) Date of publication of application: 17.12.2003
(73) Proprietor: SIGMA-TAU Industrie Farmaceutiche Riunite S.p.A., 00144 Roma (IT)
(72) Inventor: MANTOVANI, Alberto, c/o Ist. di Ricerche Farma., I-20157 Milan (IT); BOTTAZI, Barbara, c/o Ist. di Ricerche Farma., I-20157 Milan (IT); PERI, Giuseppe, c/o Ist. di Ricerche Farma., I-20157 Milan (IT); MANFREDI, Angelo, c/o Ist. di Ricerche Farma., I-20157 Milan (IT)
(74) Representative: Spadaro, Marco
(86) International application number: PCT/IT2001/000530
(87) International publication number: WO 2002/036151

(56) References cited:
- WO-A-99/32516
- P. ROVERE ET AL.: "Remnants of suicidal cells fostering systematic autoaggression: Apoptosis in the origin and maintenance of autoimmunity" ARTHRITIS & RHEUMATISM, vol. 43, no. 8, August 2000 (2000-08), pages 1663-1672, XP008013504
- Y. REN ET AL.: "Apoptosis: The importance of being eaten." CELL DEATH AND DIFFERENTIATION, vol. 5, no. 7, July 1998 (1998-07), pages 563-568, XP008013460 cited in the application
- P. ROVERE ET AL.: "The long pentraxin PTX3 binds to apoptotic cells and regulates their clearance by antigen-presenting dendritic cells." BLOOD., vol. 96, no. 13, 15 December 2000 (2000-12-15), pages 4300-4306, XP002230331 W.B.SAUNDERS COMPAGNY, ORLANDO, FL., US ISSN: 0006-4971

## Description

The present invention relates to the use of the long pentraxin PTX3 (PTX3) or one of its functional derivatives for the preparation of medicament for the prevention and treatment of autoimmune diseases.

PTX3 is expressed in various cell types (Bottazzi et Al., J., Biol. Chem. 1997, 272: 32817-32823) particularly in mononuclear phagocytes and endothelial cells, after exposure to the inflammatory cytokines Interleukin 1β (IL-1β) and Tumour Necrosis Factor alpha (TNF-α).

To date, the biological function of PTX3 has not yet been understood.

PTX3 consists of two structural domains, a N-terminal domain unrelated to any known molecule, and a C-terminal domain similar to the short pentraxins such as C-reactive protein (CRP) (Breviario F., et al., J. Biol. Chem. 267:22190, 1992).

A substantial degree of similarity has been found between human PTX3 (hPTX3) and animal PTX3s. In particular, mouse PTX3 (mPTX3) is very similar to hPTX3 in terms of DNA sequence and gene organization and location. The degree of identity between human and murine PTX3 gene is 82%, and reaches 90% if conservative substitutions are considered (Introna M., et al.: Blood 87 (1996) 1862-1872). The murine PTX3 gene is located on chromosome 3 of the mouse in a region similar to the human 3q region (q24-28), in agreement with the documented location of hPTX3 in the 3q 25 region (Introna M., et al.: Blood 87 (1996) 1862-1872).

The high degree of similarity between hPTX3 and mPTX3 sequences is a sign of the high degree of conservation of pentraxins during evolution (Pepys M.B., Baltz M.L.: Adv. Immunol. 34:141, 1983).

For a review about the pentraxins see H. Gewurz et al, Current Opinion in Immunology, 1995, 7:54-64.

The long pentraxin PTX3 is produced in tissues exposed to pro-inflammatory signals furnished both by citokynes and lipopolysaccharide (LPS).

These pro-inflammatory compounds promote the apoptotic death of cells involved in the inflammatory process and the physiological dendritic cells (DCs) maturation.

Cell death usually occurs *in vivo* during the inflammatory process.

In the organism apoptotic dying cells are removed from living tissues. During an excessive and disorderly apoptosis the huge amount of dead cells blocks the capacity of clearance of cell corps exerted by the organism. These cells accumulate in the tissue and are available for phagocytosis from non-conventional phagocytes such as immature DCs, increasing the possibility of promoting autoimmune process (Cell Death Diff. 1998; 5:563-568).

The development and maintenance of the autoimmune processes needs that autoantigen (self-antigen) presented by immature DCs are recognized as harmful (extraneous or non-self) for the organism. This recognition error happens during the removal of the apoptotic cellular corps exerted by immature DCs.

The intracellular autoantigens are modified during apoptosis, and protein esterification due to the kinases activated by the apoptotic stress produces new conformational epitopes (1998 J. Exp. Med. 187:547-560).

Cellular death and nuclear collapse are followed by autoantigen breakage made by specific proteases (caspases) 1996 J. Exp. Med. 184:765-770; 1996 J. Exp. Med. 183:1957-1964, and by the generation of nucleosomes, a major antigen in the prototypic systemic autoimmune disease, Systemic Lupus Erythematosus (SLE) (1993 J. Exp. Med 177:1367-1381; 1999; 1999 Arthritis Rheum. 42:833-843.

Some autoantigens are usually associated with the endoplasmatic reticulum and the plasmatic membranes of the cytoskeleton; these antigens are segregate during the early stage of apoptosis.

Nuclear autoantigens segregate with apoptotic corps produced during late apoptosis are expelled from the apoptotic cells 1994 J. Exp. Med. 179:1317-1330; 1997 Exp. Cell Res. 234:512-520.

Conversely, the processing of the internalized dying (apoptotic) cells yields T cells epitopes 1997 J. Immunol. 159:5391-5399.

Specific or semi-specific scavenger phagocytes which are devoid of the ability to initiate immune responses, perform most of the clearance of dying cells in vivo 1998 Cell Death Diff. 5:563-568. However, the most potent antigen presenting cells (APC) are the DCs 1997 Curr. Opin. Immunol. 9:10-16; 1998 Nature 392:245-252, these cells internalise dying cells and present epitopes derived from their processing to MHC class I- and class II-restricted T lymphocytes 1998 Nature 392:86-89; 1998 J. Immunol. 159:5391-5399; 1998 J. Exp. Med. 188:2163-2173; 1999 Nat Med 5:1249-1255; 1999 Nat Med 5:1232-1233.

DCs originate from bone marrow CD34+ progenitors 1992 Nature. 360:258-261.

DC precursors enter the blood and reach peripheral organs where they develop to immature DCs.

Immature DCs capture soluble antigens via macropinocytosis 1994 J. Exp. Med. 179:1109-1118, and particulates through phagocytosis 1993 J. Exp. Med. 178:479-488.

Primary pro-inflammatory signals like IL-1β and TNF-α promote DCs physiological maturation and antigen presentation function 1999 Curr. Opin. Immunol. 11:308-313.

The presentation of dying cell antigens from DCs is tightly regulated, since cells (by definition reservoir of "self" antigens) die continuously during the development and normal tissue turnover.

Normal cell death via apoptosis occurs in the absence of inflammatory signals causing DCs maturation.

Therefore dying cells and maturing DCs co-exist, threatening tolerance towards peripheral antigens 1999 Nat. Med. 5:1232-1233. Autoimmunity, however, does not as a rule associate with inflammation.

In physiological conditions only a low number of apoptotic cells are phagocyted by immature DCs cells.

Factor(s) in the milieu, possibly recruited by the inflammatory signals themselves, must prevent phagocytosis of dying cells by immature DCs.

Only during an excessive and disregulated inflammation, with an increased cellular death, the non-conventional phagocytes increase their phagocytotic activity, and this increased phagocytosis causes an increase of the possibility that self-antigens are recognized as non-self by the organism.

These mechanism of identification of self antigens as harmful or extraneous to the organism, possessed by immuno-competent cells, is at the base of the onset of a number of autoimmune diseases such as Systemic Lupus Erythematosus (SLE), multiple sclerosis (MS), arthritis, diabetes, thyroiditis, haemolytic anemia, atrophic orchitis, Goodpasture's disease, autoimmune retinopathy, autoimmune thrombocytopenia, myasthenia gravis, primary biliary cirrhosis, aggressive chronic hepatitis, ulcerative colitis, dermatitis, chronic glomerulonephritis, Sjogren syndrome, Reiter syndrome, miositis, systemic sclerosis and polyarthritis.

Systemic lupus erythematosus is an inflammatory systemic autoimmune disease characterized by a production of autoimmune antibody depending from T cells, endowed with high affinity.

Patients with SLE in the active phase of the disease show characteristic low plasmatic levels of C-reactive protein CRP.

In patients affected by SLE it has been found a congenital lack of the complement factor Clq (1998 Nature Genetics 19:56-59; 1998 Nature Genetics 19:3-4), factor that is recognized by PTX3 (1997 J. Biol. Chem. 272:32817-32823).

Moreover, PTX3 binds some SLE well-characterized autoantigens, such as histones and others (1990 J. Exp. Med. 172:13-18; 1997 J. Biol. Chem. 272:32817-32823).

Previous uses of PTX3 are already known, for example, WO99/32516, filed at the name of the applicant, describes the use of the long pentraxin PTX3 for the therapeutic treatment of tumoral, inflammatory and infective pathologies.

US 5767252 describes a growth factor for neuronal cells belonging to the pentraxin family (see also the cited publication). This patent relates to the neurobiology field.

To date does not exist a satisfactory cure for most of the autoimmune diseases known. A strongly perceived need for new drugs both capable of inhibiting the recognition of the self antigens as extraneous, and capable of alleviating the symptoms of these pathologies without causing further side effects still exists.

This type of inhibition determines the blockage of the onset of autoimmune diseases, which are at the base of the above-cited pathologies.

It has now surprisingly been found that the long pentraxin PTX3 is useful for preventing and alleviating the symptoms of autoimmune diseases.

In fact, the long pentraxin PTX3 is capable to bind apoptotic cells (but not living cells) preventing that these cells are available to be phagocyted by immature DCs, during an excessive or disregulated apoptosis.

As above cited, during the inflammation and increased cellular death the huge amount of death cells blocks the capacity of the organism to remove such cells, these cells accumulate into the tissues and are available for the phagocytosis made by non conventional phagocytes such as immature DCs cells, with an increase of the possibility that the autoimmune process starts.

The binding between apoptotic cells and PTX3 helps the apoptotic cells clearance made by macrophages, impeding that the apoptotic cells are phagocyted by immature DCs cells which promote autoimmune diseases.

The autoimmune diseases that can be treated according to the present invention are selected from the group consisting of Systemic Lupus Erythematosus (SLE), multiple sclerosis (MS), arthritis, diabetes, thyroiditis, haemolytic anemia, atrophic orchitis, Goodpasture's disease, autoimmune retinopathy, autoimmune thrombocytopenia, myasthenia gravis, primary biliary cirrhosis, aggressive chronic hepatitis, ulcerative colitis, dermatitis, chronic glomerulonephritis, Sjogren syndrome, Reiter syndrome, miositis, systemic sclerosis and polyarthritis.

For "long pentraxin PTX3" is intended any long pentraxin PTX3, or their functional analogous, independently from their origin (human or animal) or synthetic.

The long pentraxin preferred is the human PTX3 which sequence is described in WO99/32516.

In the following are reported some examples that illustrate the invention.

### EXAMPLE 1

### Capability of PTX3 to bind apoptotic human cells and to regulate their elimination.

### Cell types

### 1. Leukemic cells.

Human leukemia Jurkat T cells were purchased from ATCC (Rockville, MD) and grown in RPMI (Gibco BRL, Grand Island, NY) containing 100 U/ml penicillin, 0.1mg/ml streptomycin, 2 mM L-glutamine and 10% FCS (Hyclone, Logan, UT) (Tissue Culture Medium, TCM) or 1% Nutridoma-SP (Boehringer Mannhein, Germany) (TCM-nut).

### 2. Dendritic cells.

Immature human DCs derived from circulating monocytes were cultured in the presence of recombinant GM-CSF and IL-4 [(1999) J. Leuk. Biol., 66:345-349; (1997) J. Exp. Med. 185:317-328], this treatment allowed the generation of homogenous populations of immature DCs cells.

### Induction of apoptosis

Jurkat cells were propagated in TCM-nut to avoid interference by uncharacterized serum co-factors and committed to apoptosis with anti-CD95 moAb (CH-11, 100 ng/10⁶ cells) in TCM-nut at 37°C or irradiated under an U.V. source for 20". Apoptosis was verified by flow cytometry and by morphological features, as described (Arthritis Rheum. 1998; 41:205-214): after both treatment most Jurkat T cells (>95%) underwent apoptosis.

The exposure of phosphatidylserine (PS) was assessed by flow cytometry and confocal microscopy (see below) after staining with FITC-labeled Annexin V (Bender MedSystems, Prodotti Gianni, Milano, Italy) (0.5 µg/ml) for 10 minutes at room temperature in PBS containing MgCl2, 0.1 mM and CaCl2, 0.1 mM (PBS++).

DNA content was detected by flow cytometry.

In some experiments cells were committed to apoptosis by different pharmacological treatment (BAPTA-AM, 5 µM, Cycloheximide, 10 µg/ml, Staurosporin 20 µM, pyrrolidine dithiocarbamate PDTC 100 µM and 50 mM, dexamethasone, 100 nM).

Necrosis was achieved either heating cells (95°C for 5 minutes), freezing and thawing them, or by hyperhosmotic shock (10 minutes in a NaP 150 mM, pH 7.5, NaCl 1.4 M buffer).

### Proteins

Human PTX3 was purified from CHO cells stably and constitutively expressing the protein as described in J. Biol. Chem. 1997; 272:32817-32823. Biotinylation of PTX3 was performed as described EMBO J.1992; 11:813-89; biotinylated PTX3 was subsequently analyzed in the native state in 5-10% gradient PAGE as described in J. Biol. Chem. 1997; 272:32817-32823.

Purified human C-reactive protein (CRP), Serum amyloid P component (SAP) and bovine serum albumin (BSA) were purchased from Sigma (St. Louis, MO).

### Binding of PTX3 to apoptotic cells

Cells were challenged with biotinylated PTX3 (range tested 0.1-500 µg/ml) for 30 minutes at room temperature.

Cell-bound cofactors were revealed by flow cytometry after addition of FITC-conjugated streptavidin (Pierce, Rockford, IL). The fluorescence background was calculated in the presence of FITC-conjugated streptavidin only.

Linear increse of the binding was observed when increasing concentrations of biotinylated PTX3 were used, reaching a plateau at around 100 µg/ml.

The specificity of the binding was verified pre-incubating apoptotic cells with PTX3, SAP, CRP and BSA (500 µg/ml) before addition of biotinylated PTX3 (10 µg/ml), FITC streptavidin and FACS analysis.

The binding of PTX3 to dying cells was also characterized by confocal imaging: in this case stained cells were seeded on polylysine coated coverslip for 20' at RT, fixed for 15 minutes at room temperature in 4% (wt/vol) paraformaldehyde and mounted in Mowiol medium.

Confocal laser scanning microscopy was performed using a Leica TCS-NT (Leica Microsystems, Heidelberg, Germany) confocal laser scanning microscope, equipped with laser Argon/Krypton, 75 mW multiline.

Jurkat cells undergoing CD95 (FAS)-triggered apoptosis were easily identified based on nuclear characteristics such as chromatin margination, condensation and fragmentation.

### Phagocytosis

Jurkat cells at a logarithmic phase of expansion were committed to apoptosis by U.V. irradiation and-labeled with the green fluorescent aliphatic dye PKH2-GL (Sigma).

After washing cells were incubated or not with PTX3 (100 µg/ml for 30 minutes at room temperature) and co-incubated with DC(s) for 90 minutes at 37°C or at 4°C.

Both immature DCs and DCs that matured after treatment with TNF-α (1997 J. Exp. Med. 185:317-328) were used. As a control, the internalization of FITC-labeled ovalbumin (Sigma) or of green fluorescent latex beads (2 µm diameter, Polysciences, Warrington, PA) by immature and mature DCs was assessed in the presence or in the absence of PTX3. After phagocytosis DCs were incubated in PBS containing EDTA and trypsin for 15 minutes at 37°C and analyzed by flow cytometry (FACS Scan, Becton and Dickinson, San Josè, CA).

The percentages of cells that bound or internalized apoptotic cells were compared in independent samples.

### EXAMPLE 1/1

As above mentioned, Jurkat cells were challenged with biotinylated PTX3 and FITC-streptavidin, and the binding was then assessed by flow cytometry.

The results (representative of four independent experiments) reported in the following Table 1 are expressed as relative fluorescence intensity (RFI) calculated dividing the mean fluorescence intensity in the presence of PTX 3 and FITC-streptavidin and in the presence of FITC-streptavidin alone.

**TABLE 1**

| **Cells*** | **Apoptotic Treatment** | **RFI**** |
|---|---|---|
| Alive | None | 1 |
| Apoptotic Cells | Cycloheximide | 10 |
| Apoptotic Cells | PDTC | 12.3 |
| Apoptotic Cells | Dexamethasone | 17.9 |
| Apoptotic Cells | Staurosporine | 19.8 |
| Apoptotic Cells | U.V. Radiation (16 h) | 22.4 |
| Post-apoptotic Cells | U.V. Radiation (48h) | 2.8 |
| Necrotic Cells | Freezing | 7.3 |
| Necrotic Cells | Boiling | 2.4 |
| Necrotic Cells | Hyperosmotich shock | 5.6 |

| | | |
|---|---|---|
| *=Jurkat Cells; | | |
| **=Relative Fluorescence Intensity. | | |

These results shown that PTX3 acquired the ability to bind to cells undergoing later apoptosis and that recognition by PTX3 does not depend on the initiating stimuli used to trigger apoptosis. Moreover PTX3 lost the ability to bind to cells evolving toward a postapoptotic phase.

The results reported in Table 1 shown that only apoptotic cells were able to bound to biotinylated PTX3, and this is the demonstration of the specificity of the PTX 3 binding.

### EXAMPLE 1/2

As above mentioned apoptotic cells bound by PTX3 escape internalization by immature DCs.

In this experiment either immature DCs or DCs that matured after treatment with TNF-α were used.

As a control, the internalization of FITC-labeled ovalbumin or green fluorescent latex beads, by immature and mature DCs, was assessed in the presence or in the absence of PTX3.

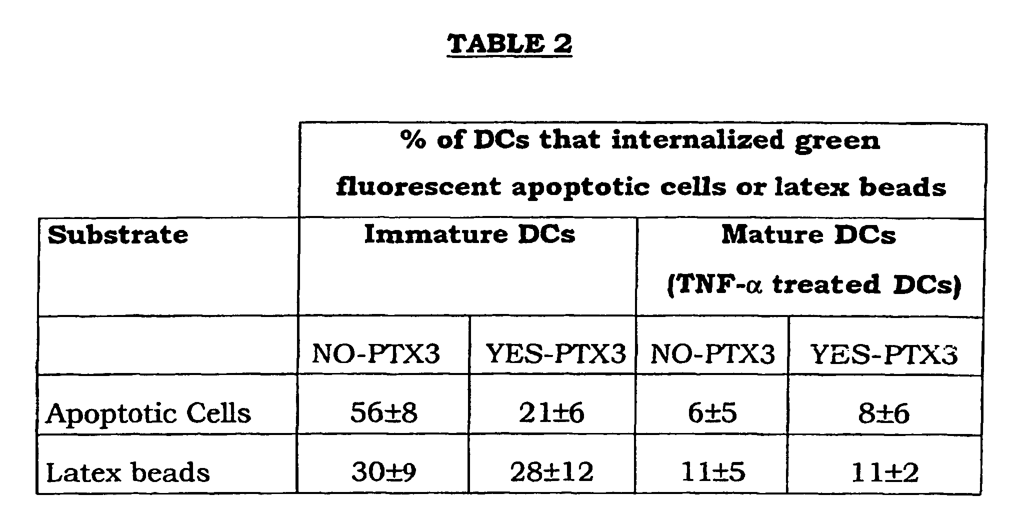

The results reported in Table 2 (mean of three separate experiment) shown that PTX3 does not influence the phagocytosis of inert substrate by immature DCs.

## Claims

1. Use of the long pentraxin PTX3 or a derivative thereof, for the preparation of a medicament for the prevention and treatment of autoimmune diseases.

2. Use according to claim 1, in which the autoimmune diseases is selected from the group consisting of Systemic Lupus Erythematosus (SLE), multiple sclerosis (MS), arthritis, diabetes, thyroiditis, haemolytic anemia, atrophic orchitis, Goodpasture's disease, autoimmune retinopathy, autoimmune thrombocytopenia, myasthenia gravis, primary biliary cirrhosis, aggressive chronic hepatitis, ulcerative colitis, dermatitis, chronic glomerulonephritis, Sjogren syndrome, Reiter syndrome, miositis, systemic sclerosis or polyarthritis.

3. Use according to claim 1 or 2, in which the long pentraxin PTX3 is the PTX3 present in nature.

4. Use according to claim 3, in which the long pentraxin PTX3 is the human pentraxin.

5. Use according to claims 1-4, in which the long pentraxin PTX3 is the PTX3 obtained synthetically.

## Patentansprüche

1. Verwendung des langen Pentraxin PTX3 oder eines Derivates davon zur Herstellung eines Medikamentes zur Vorbeugung oder Behandlung von Autoimmunerkrankungen.

2. Verwendung nach Anspruch 1, worin die Autoimmunerkrankung ausgewählt ist aus der Gruppe bestehend aus systemischem Lupus Erythematosus (SLE), Multipler Sklerose (MS), Arthritis, Diabetes, Thyroiditis, hämolytischer Anämie, atrophischer Orchitis, Goodpasture-Erkrankung, Autoimmunretinopathie, Autoimmunthrombozythopenie, Myasthenie Gravis, primärer Leberzhirrose, aggressiver chronischer Hepatitis, ulcerativer Colitis, Dermatitis, chronischer Glomerulonephritis, Sjogrensyndrom, Reitersyndrom, Miositis, systemischer Sklerose oder Polyarthritis.

3. Verwendung nach Anspruch 1 oder 2, worin das lange Pentraxin PTX3 das in der Natur vorhandene PTX3 ist.

4. Verwendung nach Anspruch 3, worin das lange Pentraxin PTX3 das menschliche Pentraxin ist.

5. Verwendung nach den Ansprüchen 1 bis 4, worin das lange Pentraxin PTX3 das synthetisch erhaltene PTX3 ist.

## Revendications

1. Utilisation de la pentraxine PTX3 sous forme longue ou d'un de ses dérivés, pour la préparation d'un médicament destiné à la prévention et au traitement de maladies auto-immunes.

2. Utilisation selon la revendication 1, dans laquelle les maladies auto-immunes sont choisies dans le groupe constitué du lupus érythémateux disséminé (LED), de la sclérose en plaques (SEP), de l'arthrite, du diabète, de la thyroïdite, de l'anémie hémolytique, de l'orchite atrophique, du syndrome de Goodpasture, de la rétinopathie auto-immune, de la thrombocytopénie auto-immune, de la myasthénie, de la cirrhose biliaire primitive, de l'hépatite chronique agressive, de la colite néphrétique, de la dermatite, de la glomérulonéphrite chronique, du syndrome de Sjogren, du syndrome de Reiter, de la myosite, de la sclérose systémique ou de la polyarthrite.

3. Utilisation selon la revendication 1 ou 2, dans laquelle la pentraxine PTX3 sous forme longue est la PTX3 présente dans la nature.

4. Utilisation selon la revendication 3, dans laquelle la pentraxine PTX3 sous forme longue est la pentraxine humaine.

5. Utilisation selon l'une des revendications 1 à 4, dans laquelle la pentraxine PTX3 sous forme longue est la PTX3 obtenue par synthèse.
